# EUROPEAN PATENT APPLICATION

(11) **EP 3 878 397 A1**
(43) Date of publication of application: **15.09.2021**
(21) Application number: 20199523.0
(22) Date of filing: 01.10.2020
(51) Int. Cl.: A61B 90/70, F16L 25/14

(54) **CONNECTION ADAPTER FOR FLUSHING DEVICES**

(30) Priority: 01.10.2019 DE 102019126425
(71) Applicant: MMM Münchener Medizin Mechanik GmbH, 82152 Planegg (DE)
(72) Inventor: Eibl, Robert, 82152 Planegg (DE); Weitze, Wolfram, 86899 Landsberg am Lech (DE); Aghajani, Farzad, 82166 Gräfelfing (DE)
(74) Representative: Wittmann, Ernst-Ulrich

(57) **Abstract**

A connection adapter for flushing devices comprising: a first adapter part, configured to be connected with at least one flushing media outlets of a flushing device; a second adapter part capable of moving with respect to the first adapter part; and at least one biasing element, biasing the second adapter part in a predetermined direction with respect to the first adapter part, wherein the first adapter part provides at least partially a form-fit connection with the second adapter part, the second adapter part can be arranged in at least a first and a second position along the predetermined direction, and when the second adapter part is placed in the first position the connection adapter at least partially closes the flushing media outlet of the flushing device, and when the second adapter part is placed in the second position, the connection adapter opens the flushing media outlet of the flushing device, and, together with the first adapter part, forms a receiving chamber for receiving an item to be flushed.

## Description

Disinfection of reusable tools, devices and equipment in environments requiring high standards of hygiene, such as e.g. medical facilities, food manufacturing facilities, tattoo parlours or clean rooms, plays a fundamental role in the schedule of facilities maintenance.

An efficient and reliable process is not only advantageous in terms of the end result of the disinfection procedure, but determines financial repercussions when said process need no further repetition and can be safely run by the personnel in reliable and time-saving manners.

Disinfection machinery are nowadays equipped with a number of containers, trays, accommodating receptacles and similar shelves or structural arrangements to receive special as well as ordinary items to render the disinfection process as effective as possible for different types and sizes and shapes of such tools, devices and general equipment.

A special challenge is represented by hollow instruments, tools, surgical items, requiring either pressurized medium to be flushed through their cavities and/or the addition of special disinfection additives to avoid capillary effect and to ensure that every internal surface, crevice and corner is exposed to the disinfection medium.

In the more advanced machinery, each special tool, device or instrument is specifically connected to flushing media outlets, either e.g. on a removable/portable flushing device, or on a tray, or on a specifically equipped shelve of some sort, to ensure that a leakage-proof connection is established, and no solution of continuity can be devised between the flushing outlet and the item to be flushed, in order to avoid wastage of flushing medium, namely disinfecting or flushing medium, at the interconnecting junctions and related drops in pressure, both circumstance that can negatively impact the disinfection process and its results.

In fact a disinfection system includes a circuit through which pressurized medium might be employed, in order to reach e.g. the hollow items, tools or devices connected through the flushing media outlets; to perform at the desired pressure, the circuit must be closed or at least partially closed, which means that the pressure within the system can be maintained within tolerance values that can still allow the system to perform as intended. In these conditions, generally sudden pressure drops are not desirable for a correct completion of a disinfection procedure.

The flushing media outlets present on a given device or support, e.g. a removable/portable flushing device, a tray, a specifically equipped shelve of some sort, especially within a disinfection machine, may not need using at all times, therefore those which are not in use during a particular cycle should be conveniently blocked or deactivated. This action should be carried out to ensure the above conditions, namely wastage of disinfecting medium and unwanted or uncontrolled and sudden decrease in pressure to be avoided, so that the whole procedure is not negatively affected in terms of intermediate and end results, or interrupted altogether for system malfunction, or in need of undergoing unduly repetitions.

This task may reveal particularly daunting given the large variety of outlets and connectors, and the necessity to save time, personnel costs and human or machine errors to fit certain clearly bespoken connectors and media outlets.

Some attempts in the prior art include either relatively simple or complex solutions, including standard threaded stoppers for the flushing media outlets, which are not ideal to either pressure resistance nor for preventing contamination in or around unflushed recesses and grooves.

In certain cases, the structural and mechanical features of such solutions are not ideal for both functionality and ease of connecting and disconnecting to a flushing media outlet by both trained and in-training personnel. This means that some of these solutions may, for example, deform under pressure, collect contaminants in their recesses and grooves, require long times for connecting and disconnecting, provide poor reliability upon reconnection, etc..

The present connection adapter for flushing devices, as described in the present invention, at least partially addresses the disadvantages of the prior art.

In particular, according to the present invention, a connection adapter for flushing devices comprises: a first adapter part, configured to be connected with at least one flushing media outlets of a flushing device; a second adapter part capable of moving with respect to the first adapter part; and at least one biasing element, biasing the second adapter part in a predetermined direction with respect to the first adapter part.

Moreover, according to the connection adapter of the present invention, the first adapter part provides at least partially a form-fit connection with the second adapter part, the second adapter part can be arranged in at least a first and a second position along the predetermined direction, and when the second adapter part is placed in the first position the connection adapter at least partially closes the flushing media outlet of the flushing device.

Further, when the second adapter part is placed in the second position, the connection adapter opens the flushing media outlet of the flushing device, and, together with the first adapter part, forms a receiving chamber for receiving an opening of an item to be flushed.

A flushing media outlet is a working element in a flushing device, a support tray, a tray, a disinfection machine, etc... Through the flushing media outlet, special disinfecting or flushing media are let out to a given pressure in order to flush hollow items, e.g. reusable medical items, surgical or food-grade reusable tools. Flushing media encompass a number of liquids, fluids, suspensions and mixed flushable media, e.g. water- or vapour-based mixtures, solutions and dispersions or pure solvents or mixtures thereof.

A disinfection procedure involves the removal of pathogens from a reusable item that will have some contact with a human, animal or clean environment where the threat of infection may be high. Any item or tool that has an ordinary or extraordinary level of pathogens, microorganisms capable of causing infections, including medical tools, medical or surgical equipment, needles and food-grade items, tools or devices, requires at some point to undergo a disinfection cycle, in order to reduce the number of pathogens to a minimum, so that the recirculation of such items does not pose an infection threat.

Flushing media through outlets in a flushing device, a support tray, a tray, a disinfection machine, etc... includes flushing disinfection media for the purpose of disinfecting e.g. reusable medical or surgical instruments, items and tools, in particular hollow items, e.g. including cavities, recesses and capillaries.

A connection adapter according to some embodiments of the present invention is intended e.g. as a complement tool in particular for media outlets of a flushing device. Such adapter adapts to size and shape of the connector and is able to form a receiving chamber for any flushing media outlet, together with an item or tool or device to be e.g. flushed, cleaned, disinfected or any combination thereof.

A first adapter part is an element of the connection adapter and guarantees e.g. the stability of the adapter by connecting to the flushing media outlet; a first adapter part may be intended as a stationary reference frame for the second adapter part to be capable of moving with respect to it. The second adapter part forms the receiving chamber where a tool, item or device to be flushed is received.

A biasing element according to the present invention is an element able to bias the first adapter part with respect to the second adapter part in a predetermined direction, to the end, according to how it is arranged and its structural properties, of eventually providing resistance to performing a predetermined movement and/or to maintaining a predetermined position. This allows, amongst other uses, to form the receiving chamber in combination with first and second adapter parts.

In the meaning of the present invention, biasing is intended, among others, as e.g. pre-stressing, compressing, pre-loading, stretching or twisting, or determine, by mechanical or other means, a deformation or stress of the biasing element; in this way, potential energy is stored in the biasing element, in particular elastic potential energy, which may then be converted into motion or displacement, or both, once the mechanical or other stressing means are removed.

Otherwise, until the stressing means are in place, such biasing element is capable of performing other types of work, and, for example, holding in place one or more of the adapter parts with respect to a predefined position.

According to an embodiment of the present invention, when the second adapter part of the connection adapter is arranged in the first position and the connection adapter at least partially closes the flushing media outlet of the flushing device, a perfusion flow of the flushing media is allowed to flow through the flushing media outlet between the flushing media outlet and the second adapter part.

The at least partially closed position in the meaning of the present invention refers to a position wherein a flow of disinfecting medium is still allowed through the flushing media outlet, but said outlet is considered as not in use for extensive flushing activities and is closed with respect to a connection with an item or a tool to be flushed.

Therefore, while the outlet is not necessarily physically blocked or locked with respect to a flow of disinfecting medium, in the at least partially closed position it is not intended to be receiving an item or a tool to be flushed.

The perfusion flow, allowed to run between the second adapter part in the first position and the flushing media outlet of the flushing device, allows for the main pressure in the system to be maintained without a sudden decrease, and, at the same time, the flow of medium ensures the flushing of the contact surfaces, so that no contaminants are left in any recess, corner, groove or namely in any place flushed by the flow, and especially the flushing media outlet.

This guarantees that the next time the flushing media outlet is employed in connection with a tool, or item, to be flushed, the media outlet is in disinfected conditions and without any debris from the disinfection cycles or from previous uses.

Perfusion according to the present invention is the passage of a small volume of fluid, or in this case of flushing medium, through a hollow path, or guide, or pipe, of a given cross-section. In the meaning of the present invention, a small flow is defined within the ranges given in the following paragraphs, and in particular with comparison to the large, or larger, volumetric flow intended to be the operational flow that circulates in a flushing system and is used to run the disinfection processes and cycles.

In particular, such perfusion volumetric flow is intended as running through the same channels, paths and pipes as the fully operational, large, or larger, volumetric flow in the flushing device, when the second adapter part is in the first position, and wherein the first position is associated with an at least partially closed flushing media outlet, which is not in connection with a tool, item or device to be flushed.

According to the present invention, the perfusion flow in the above connection adapter is in the range from 1 to 40 ml/s, and preferably in the range from 5 and 30 ml/s, and more preferably in the range from 10 and 20 ml/s, and in particular in a range greater than 0 and up to 20%, and preferably in the range between 0,05 and 10% of the regular flushing media flow.

This volumetric flow enables the flushing activity to be maintained to the regular operational levels and prevents accumulation of debris, contaminants and infection agents with respect to the flushing media outlet.

According to embodiments of the present invention, the first adapter part is made of a material belonging to one of suitable groups of plastics, e.g. polyoxymethylen copolymer, polyethylene terephthalate (PETE or PET), high-density polyethylene (HDPE), polyvinyl chloride (PVC), low-density polyethylene (LDPE), polypropylene (PP), or mixtures thereof, e.g. polycarbonate, polylactide, acrylic, acrylonitrile butadiene, styrene, fiberglass, nylon; rubbers, e.g. neoprene, polychloroprene, nitrile, silicone, polysiloxane, ethylene propylene diene monomer (EPDM), natural gum, fluoroelastomer, natural rubber, styrene butadiene, santoprene, thermoplastic vulcanizate, epychlorohydrin, isobutyl isoprene, isoprene, latex, latex-free thermoplastic elastomer, thermoplastic elastomer, chlorosulfonated polyethylene; metals, e.g. manganese, nickel, chromium, molybdenum, boron, titanium, vanadium, tungsten, cobalt, and niobium, non-metals or alloys, e.g. steel, stainless steel, carbon steel, alloy steel; and/or a combination thereof.

According to embodiments the present invention, the second adapter part is made of a material belonging to one of suitable groups of plastics, e.g. polyoxymethylen copolymer, polyethylene terephthalate (PETE or PET), high-density polyethylene (HDPE), polyvinyl chloride (PVC), low-density polyethylene (LDPE), polypropylene (PP), or mixtures thereof, e.g. polycarbonate, polylactide, acrylic, acrylonitrile butadiene, styrene, fiberglass, nylon; rubbers, e.g. neoprene, polychloroprene, nitrile, silicone, polysiloxane, ethylene propylene diene monomer (EPDM), natural gum, fluoroelastomer, natural rubber, styrene butadiene, santoprene, thermoplastic vulcanizate, epychlorohydrin, isobutyl isoprene, isoprene, latex, latex-free thermoplastic elastomer, thermoplastic elastomer, chlorosulfonated polyethylene; metals, e.g. manganese, nickel, chromium, molybdenum, boron, titanium, vanadium, tungsten, cobalt, and niobium, non-metals or alloys, e.g. steel, stainless steel, carbon steel, alloy steel; and/or a combination thereof.

In the meaning of some embodiments of the present invention, the chosen materials that constitute the connection adapter, and partially listed above, are considered suitable with respect to their function in a given embodiment, and for withstanding given operational conditions e.g. in a disinfection chamber, such as, for example, temperature, humidity, and pressure. In certain embodiments of the present invention, the first adapter part is made of stainless steel and the second adapter part of polyoxymethylene copolymer. Nevertheless, according to embodiments that a skilled person can derive from the present applications, any of the indicated suitable materials can be used, according to a specific application.

According to an embodiment of the present invention, the biasing element is a helical spring. This allows for employing the potential energy stored through compressing the helical spring, and inducing elastic deformation to perform work, such as, for example, to move the second adapter part relatively to the first adapter part and for enabling the combination of the two adapter part to hold a given position.

According to an embodiment of the present invention, the biasing element has a working length in the range from 1 to 30 mm, and preferably in the range from 5 to 27 mm, and more preferably in the range from 10 to 15 mm, and preferably in a ratio of 2:1, or 5:1, or 5:2 with a cross-sectional dimension of the receiving chamber.

According to other embodiments of the present invention, the biasing element has an elastic modulus in the range from 50 to 250 N/mm², and preferably in the range from 70 to 200 N/mm² and more preferably of 185 N/mm²; moreover, in the meaning of some embodiments of the present invention, the biasing element has a shear modulus in the range from 20 to 100 kN/mm² and preferably in the range from 50 to 80 kN/mm² and more preferably of 70 kN/mm². Further, the biasing element according to an embodiment of the present invention has a bulk modulus in the range from 50 to 200 GPa and preferably in the range from 70 to 180 GPa and more preferably of 160 GPa.

In the meaning of the present invention, a biasing element, characterized by the above physical parameters, is able to carry out a work that determines the displacement or the holding in place of one or of both the adapter parts, with respect to a flushing media outlet, as received in the receiving chamber formed when the second adapter part is in the second position with respect to the first adapter part.

According to an embodiment of the present invention, the one or more flushing media outlets have a regular polygonal geometry.

The flushing media outlets may have any shape, according to the specific instrument, tool, item or device to be flushed with disinfecting medium; nevertheless, it is a favourable feature from a construction and manufacturing point of view that some media outlet have a regular polygonal geometry for receiving objects featuring similarly regularly shaped connectors.

According to an embodiment the present invention, the at least one flushing media outlets are concentric to one portion of the second adapter part, when the second adapter part is in the second position.

In the meaning of the present invention, a favourable symmetry, in the relative shape or geometry of a flushing media outlet or of the receiving chamber formed when the second adapter part is in the second position, is a central symmetry, so that, e.g. a center of the receiving chamber is in line with a center of the flushing media outlet.

According to an embodiment of the present invention, the second adapter part slides along the first adapter part in a direction perpendicular to a symmetry axis of the first adapter part.

Sliding in the meaning of the present invention is intended as the structural ability of one adapter part to move along a direction of movement, in both verses, without necessarily engaging with any of the surfaces of another adapter part, unless the engagement is wanted or the profile of each part is so designed to allow for a temporary or permanent engagement. Sliding is also intended according to the present invention as a movement of one adapter part relative to the other that does not necessarily involve friction or contact between the parts, unless the parts are designed to slide while in close contact. Sliding may also involve friction, according to the present invention in the event that the walls of each adapter, that are able to slide relatively to each other, are in contact or close contact.

According to an embodiment of the present invention, the second adapter part slides along the second adapter part in a direction parallel to a symmetry axis of the first adapter part.

Depending on the shape of the adapter parts, it is a favourable arrangement in manufacturing terms that the sliding of a second adapter part happens along a direction, which is parallel to a symmetry axis of the first adapter part.

According to an embodiment of the present invention, the second adapter part turns/twists with respect to the outer profile/perimeter/circumference of the first adapter part in a plane that is orthogonal to a symmetry axis of the first adapter.

Depending on the shape and geometry of the adapter parts, their mutual or relative position can be altered in appropriate ways that profit from the particular structural arrangements in accordance with said shape and geometry. Therefore, in arrangements where profiles are matching in shape so that, e.g. both have clearance for movement in a given direction, along a given path or else, and the relative position of the adapter parts includes that portions or surfaces or walls of one adapter part can slide relative to another adapter part, a possible path or direction of movement of one part belong to a plane that is orthogonal to a symmetry axis of the other part to ensure a general flexibility of the system.

According to a further embodiment of the present invention, the second adapter part is free to twist with respect to the outer profile/perimeter/circumference of the first adapter part.

The ability of providing a freedom of absolute motion of the connection adapter according to the present invention, and/or relative motions of a first adapter part to the second adapter part, and vice-versa, is inherent to the design of said connection adapter and of its parts, so that a twisting movement, for example, among others, is possible if structural requirements for mounting the adapter on a particular flushing device and with respect to a given flushing media outlet need be fulfilled. Twisting in the meaning of the present invention includes a rotational motion of one adapter part to another adapter part and a form of at least partial relative rotational symmetry of the adapter parts to add flexibility to the system; it also includes, in combination with the features of other embodiments, the ability of one adapter part to bend or deform or being bent upon, e.g., the deployment of the biasing element, with respect to a fixed reference frame or to another adapter part.

According to another embodiment of the present invention, the first and second adapter parts have one or more of openings, such as slits and holes, grooves, and/or partial openings, such as hinged die-cuts etc....

In the meaning of the present invention, structural features of an adapter part including e.g. openings, slits, holes, partial openings, die-cuts and hinged die-cuts determine at least one possibility of forming a receiving chamber while the adapter parts are biased by the biasing element. Said openings therefore help forming the receiving chamber to the end of receiving the flushing media outlet in connection with an item to be flushed and at the same time help achieving the effect of at least partially closing the flushing media outlet when not deployed, and namely when not in connection with an item to be flushed.

Openings such as those considered above can also be included in the structural features of an adapter part to facilitate the dispersion of contaminants, debris and residues from the disinfection procedures in the flushing device or disinfection machine.

According to an embodiment of the present invention, the first and second adapter parts have at least one contact surface.

According to an embodiment of the present invention, moving includes sliding, twisting, swivelling etc...

Besides the definitions given above, swivelling includes the movement of one part of the connection adapter according to the present invention along a connection point, or line or surface, with respect to another part, so that a coupling can be maintained between two adapter parts that allows the movement of one part with respect to the other, while said other part is kept stationary.

According to an embodiment of the present invention, the first adapter part has a form-fit connection with the flushing media outlet.

In this way, the movements of one adapter part relative or with respect to the other can be guided or controlled or directed through a particular shape adaptation of a degree of interlocking of the adapter parts to perform a particular function, be it the holding of a position or the moving relative to one another along a given direction.

According to an embodiment of the present invention, the second adapter part is capable of locking into the first adapter part.

According to this embodiment, the adapter parts are enabled by locking means or mechanisms to maintain a desired position in order to perform a given function, such as forming a receiving chamber or rather at least partially closing the flushing media outlet to allow perfusion and maintenance of the operational parameters of a flushing device during a disinfection procedure.

According to an embodiment of the present invention, the first and second adapter parts have one or more of grooves and/or protrusions.

This allows for the formation and maintenance of a mechanical locking system and a mechanical and form-fit connection of the adapter parts.

Moreover, in another embodiment of the invention, the second adapter part locks in the first adapter part when the second adapter part is in the second position; further to the previous embodiments of the present invention, the second adapter part locks in the first adapter part in a position intermediate between the first and the second position. According to a further embodiment of the present invention, the second adapter part is arranged in a position intermediate between the first and second positions and a plurality of positions intermediate between the first and second positions is provided.

In the above embodiments examples are provided of relative positioning of the first and second adapter parts, wherein said arrangements enable the functions of: forming a receiving chamber; and of at least partially closing the flushing media outlet to allow perfusion and maintenance of the operational parameters of a flushing device during a disinfection procedure, to be performed.

The connection adapter according to the present invention is intended for use in e.g. a fixed or portable disinfection machine, facility and/or device or tool; moreover, said connection adapter, according to the present invention, is also intended for use in the flushing of hollow tools in the medical, technological, manufacturing and scientific devices industry, e.g. in scientific laboratories, clean room environments, such as e.g. space technology or precise manufacturing grade clean rooms, as well as in the food and food manufacturing industry.

The connection adapter according to the present invention is intended for use with a variety and/or plurality of adapters for flushing media outlets.

When taken alone or in combination or arranged in an array, a plurality of connection adapters and a variety of adapters or connectors for flushing media outlet can be operated.

Further aspects of the present invention are provided in the following detailed description, featuring possible embodiments of the invention in the guise of exemplary embodiments. It is worth noticing that any modifications or additions that can be derived directly from the person skilled in the art are covered by these examples. In particular, it is noted that such examples or preferred embodiments are not intended, nor formulated, to restrict the scope of protection of the present application.

The following figures captions and descriptions refer to schematics, which constitute part of the specification and illustrate several aspects of the present invention; taken together with the description, the figures help explaining certain principles of the invention.
**Fig. 1****:** Front view of an embodiment of the connection adapter of the present invention, in a first position, and wherein the biasing element is not deployed. In the figure some symmetry axis are shown for representative purposes only.
**Fig. 2****:** Three-dimensional view of an embodiment of the connection adapter of the present invention in a first position.
**Fig. 3****:** Front view of an embodiment of the connection adapter of the present invention, in a second position, and wherein the biasing element is deployed.
**Fig. 4****:** Three-dimensional view of an embodiment of the connection adapter of the present invention in a second position.

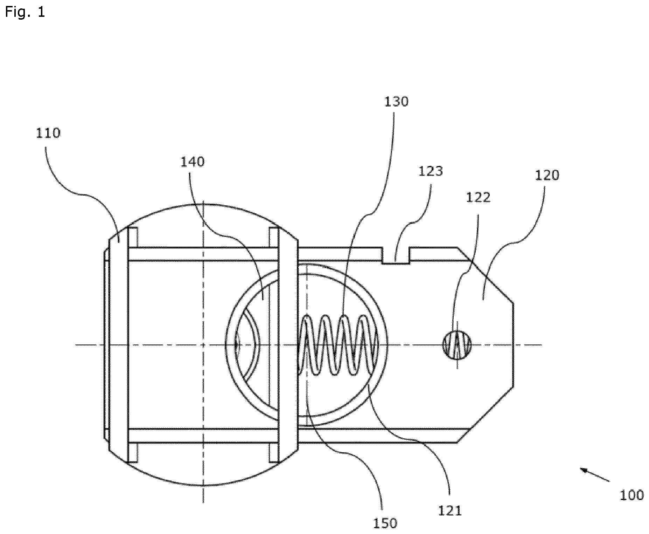

Figure 1 shows a front-view of an embodiment of the connector adapter 100 of the present invention in a first position, or at least partially closed position. In Figure 1 in particular the front-view of a first adapter part 110 is shown, together with the front-view of a second adapter part 120. A biasing element 130 is shown throughout an opening 121 in the second adapter part 120. A cross-section dimension 150 of the receiving chamber 140 formed in the second adapter part 120 by the opening 121 is also shown. Figure 1 additionally shows a hole 122 in the second adapter part to be used to flush through debris, residues and facilitate the overall disinfection procedure maintaining the components of the adapter clean and free from contaminants. Moreover, Figure 1 shows a groove 123 that is used to lock the second adapter part 120 to a portion of the first adapter part 110, when the connection adapter is operated in the second position. The figure is used purely in an explicative way and it does not directly suggest dimensions or sizes or proportions of the objects on scale or otherwise.

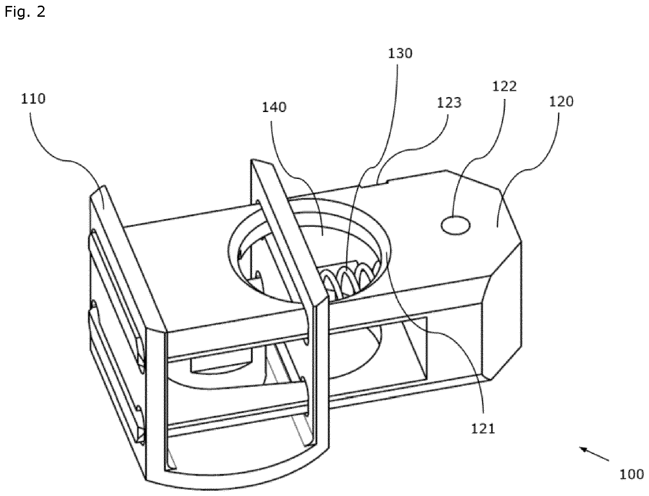

Figure 2 shows a three-dimensional view of the connector adapter 100 according to an embodiment of the present invention. In particular, specific shapes of a first adapter part 110 and of a second adapter part 120 can be seen, for the purpose of showing an example of how to carry out the invention. Moreover a particular embodiment of the biasing element 130 in the form on a helical spring can be seen throughout the receiving chamber 140, formed through the opening 121 in the second adapter part 120. Figure 2 additionally shows in a three-dimensional view a hole 122 in the second adapter part to be used to flush through debris, residues and facilitate the overall disinfection procedure maintaining the components of the adapter clean and free from contaminants. Moreover, Figure 2 shows a groove 123 that is used to lock the second adapter part 120 to a portion of the first adapter part 110, when the connection adapter is operated in the second position. The figure is used purely in an explicative way and it does not directly suggest dimensions or sizes or proportions of the objects on scale or otherwise.

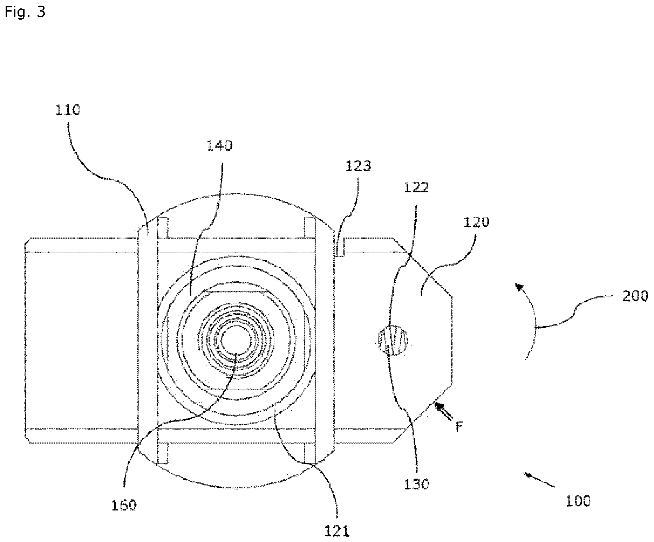

Figure 3 shows a front view of an embodiment of the connector adapter 100 of the present invention in a second position, or open position. In Figure 3 in particular the top-view of a first adapter part 110 is shown, together with the top-view of a second adapter part 120. Figure 3 additionally shows a hole 122 in the second adapter part to be used to flush through debris, residues and facilitate the overall disinfection procedure maintaining the components of the adapter clean and free from contaminants. A biasing element 130 is shown throughout the hole 122 in the second adapter part 120, and wherein the biasing element is deployed. Moreover, Figure 3 shows a groove 123 that is used to lock the second adapter part 120 into a portion of the first adapter 110, as the connection adapter is operated in the second position. To lock properly the system as shown in Figure 3, the second adapter part 120 is pressed on the point of application of force F along the direction 200 and tilted, to bias the biasing element 130, so that the groove 123 engages with a portion of the first adapter part 110, therefore holding the second adapter part in the second or open position. In the open position as shown in Figure 3 it is possible to see the flushing media outlet 160 being received in the receiving chamber, and in particular the male-taper fitting of a standardized system for leak-proof connection such as a luer taper, wherein the matching female part on medical and laboratory instruments can be inserted. As the receiving chamber 140 is formed by biasing the biasing element 130 to hold the second adapter part 120 in the second position, the flushing media outlet can be connected to an item, device or instrument to be flushed using flushing or disinfecting media. The figure is used purely in an explicative way and it does not directly suggest dimensions or sizes or proportions of the objects on scale or otherwise.

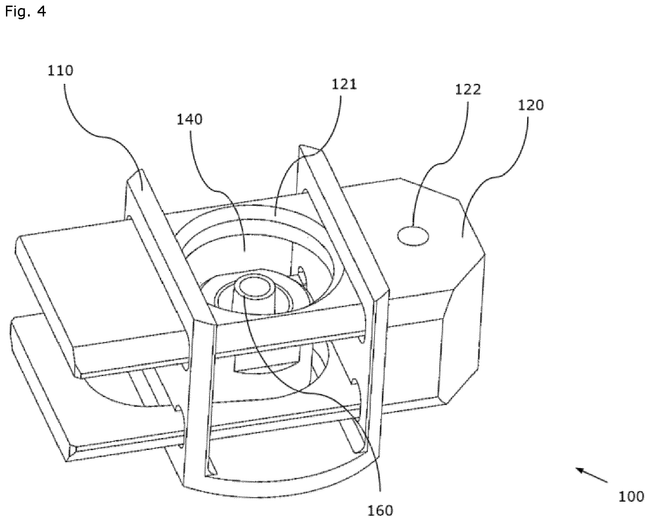

Figure 4 shows a three-dimensional view of the connector adapter 100 according to an embodiment of the present invention in a second position. In particular, specific shapes of a first adapter part 110 and of a second adapter part 120 can be seen, for the purpose of showing an example of how to carry out the invention. In the open position of the connection adapter shown in Figure 4 it is possible to see the flushing media outlet 160 being received in the receiving chamber, and in particular the male-taper fitting of a standardized system for leak-proof connection such as a luer taper, wherein the matching female part on medical and laboratory instruments can be inserted. As the receiving chamber 140 is formed by biasing the biasing element 130 to hold the second adapter part 120 in the second position, the flushing media outlet can be connected to an item, device or instrument to be flushed using flushing or disinfecting media. Figure 4 additionally shows a hole 122 in the second adapter part to be used to flush through debris, residues and facilitate the overall disinfection procedure maintaining the components of the adapter clean and free from contaminants. In the second position of the connection adapter shown in Figure 4, the second adapter part 120 is constructed to lock into a portion of the first adapter part 110, while the biasing element is deployed, for the connector to be held in the second position. The figure is used purely in an explicative way and it does not directly suggest dimensions or sizes or proportions of the objects on scale or otherwise.

**List of elements**

| | |
|---|---|
| 100 connection adapter | 130 biasing element |
| 110 first adapter part | 140 receiving chamber |
| 120 second adapter part | 150 cross-section of the receiving chamber |
| 121 opening | 160 flushing media outlet |
| 122 hole | 200 direction of tilting of the second |
| 123 groove | adapter part |
| F point of application of force F to the second adapter part to bias the biasing | element |

## Claims

1. A connection adapter (100) comprising:
a first adapter part (110) configured to be connected with at least one flushing media outlets (160) of a flushing device;
a second adapter part (120) capable of moving with respect to the first adapter part; and
at least one biasing element (130), biasing the second adapter part in a predetermined direction with respect to the first adapter part,
wherein the first adapter part provides at least partially a form-fit connection with the second adapter part,
the second adapter part can be arranged in at least a first and a second position along the predetermined direction, and
when the second adapter part is placed in the first position the connection adapter at least partially closes the flushing media outlet of the flushing device, and
when the second adapter part is placed in the second position the connection adapter opens the flushing media outlet of the flushing device, and, together with the first adapter part, forms a receiving chamber (140) for receiving an item to be flushed.

2. A connection adapter (100) according to claim 1, wherein, when the second adapter part (120) is placed in the first position and the connection adapter at least partially closes the flushing media outlet (160) of the flushing device, a perfusion flow of the flushing media is allowed to flow through the flushing media outlet between the flushing media outlet and the second adapter part.

3. A connection adapter (100) according to claim 2, wherein the perfusion flow is in the range from 1 to 40 ml/s, and preferably in the range from 5 and 30 ml/s, and more preferably in the range from 10 and 20 ml/s, and in particular in a range greater than 0 and up to 20%, and preferably in the range between 0,05 and 10% of the regular flushing media flow.

4. A connection adapter (100) according to claims 1 to 3, wherein the first adapter part (110) is made of a material belonging to one of suitable groups of plastics, e.g. polyoxymethylen copolymer, polyethylene terephthalate, high-density polyethylene, polyvinyl chloride, low-density polyethylene, polypropylene, or mixtures thereof, e.g. polycarbonate, polylactide, acrylic, acrylonitrile butadiene, styrene, fiberglass, nylon; rubbers, e.g. neoprene, polychloroprene, nitrile, silicone, polysiloxane, ethylene propylene diene monomer, natural gum, polysaccharide, fluoroelastomer, natural rubber, styrene butadiene, santoprene, thermoplastic vulcanizate, epychlorohydrin, isobutyl isoprene, isoprene, latex, latex-free thermoplastic elastomer, thermoplastic elastomer, chlorosulfonated polyethylene; metals, e.g. manganese, nickel, chromium, molybdenum, boron, titanium, vanadium, tungsten, cobalt, and niobium, non-metals or alloys, e.g. steel, stainless steel, carbon steel, alloy steel; and/or a combination thereof.

5. A connection adapter according to any of claims 1 to 4, wherein the second adapter part (120) is made of a material belonging to one of suitable groups of plastics, e.g. polyoxymethylen copolymer, polyethylene terephthalate, high-density polyethylene, polyvinyl chloride, low-density polyethylene, polypropylene, or mixtures thereof, e.g. polycarbonate, polylactide, acrylic, acrylonitrile butadiene, styrene, fiberglass, nylon; rubbers, e.g. neoprene, polychloroprene, nitrile, silicone, polysiloxane, ethylene propylene diene monomer, natural gum, polysaccharide, fluoroelastomer, natural rubber, styrene butadiene, santoprene, thermoplastic vulcanizate, epychlorohydrin, isobutyl isoprene, isoprene, latex, latex-free thermoplastic elastomer, thermoplastic elastomer, chlorosulfonated polyethylene; metals, e.g. manganese, nickel, chromium, molybdenum, boron, titanium, vanadium, tungsten, cobalt, and niobium, non-metals or alloys, e.g. steel, stainless steel, carbon steel, alloy steel; and/or a combination thereof.

6. A connection adapter according to any of claims 1 to 5, wherein the biasing element (130) is a helical spring.

7. A connection adapter according to any of claims 1 to 6, wherein the biasing element (130) has a working length in the range from 1 to 30 mm, and preferably in the range from 5 to 27 mm, and more preferably in the range from 10 to 15 mm, and preferably in a ratio of 2:1, or 5:1, or 5:2 with a cross-sectional dimension (150) of the receiving chamber (140).

8. A connection adapter (100) according to any of claims 1 to 7, wherein the biasing element (130)has an elastic modulus in the range from 50 to 250 N/mm², and preferably in the range from 70 to 200 N/mm² and more preferably of 185 N/mm² .

9. A connection adapter (100) according to any of claims 1 to 8, wherein the biasing element (130) has a shear modulus in the range from 20 to 100 kN/mm² and preferably in the range from 50 to 80 kN/mm² and more preferably of 70 kN/mm².

10. A connection adapter (100) according to any of claims 1 to 9, wherein the biasing element (130) has a bulk modulus in the range from 50 to 200 GPa and preferably in the range from 70 to 180 GPa and more preferably of 160 GPa.

11. A connection adapter (100) according to any of claims 1 to 10, wherein the one or more flushing media outlets (160) have a regular polygonal geometry.

12. A connection adapter (100) according to any of the preceding claims, wherein the at least one flushing media outlets (160) are concentric to one portion of the second adapter part (120), when the second adapter part is in the second position.

13. A connection adapter (100) according to any of the preceding claims, wherein the second adapter part (120) slides along the first adapter part (110) in a direction perpendicular to a symmetry axis of the first adapter part.

14. A connection adapter (100) according to any of the preceding claims, wherein the second adapter part (120) slides along the first adapter part (110) in a direction parallel to a symmetry axis of the first adapter part.

15. A connection adapter (100) according to any of the preceding claims, wherein the second adapter part (120) turns/twists with respect to the outer profile/perimeter/circumference of the first adapter part in a plane that is orthogonal to a symmetry axis of the first adapter part.

16. A connection adapter (100) according to any of the preceding claims, wherein the second adapter part (120) is free to twist with respect to the outer profile/perimeter/circumference of the first adapter part.

17. A connection adapter (100) according to any of the preceding claims, wherein the first and second adapter parts have one or more of openings (121), such as slits and holes (122), grooves (123), and/or partial openings, such as hinged die-cuts etc..

18. A connection adapter (100) according to any of the preceding claims, wherein the first adapter part (110) and second adapter part (120) have at least one contact surface.

19. A connection adapter (100) according to any of the preceding claims, wherein moving includes sliding, twisting, swivelling etc..

20. A connection adapter (100) according to any of the preceding claims, wherein the first adapter part (110) has a form-fit connection with the flushing media outlet (160) .

21. A connection adapter (100) according to any of the preceding claims, wherein the second adapter part (120) is capable of locking into the first adapter part (110).

22. A connection adapter (100) according to any of the preceding claims, wherein the first adapter part (110) and the second adapter part (120) have one or more of grooves (123) and/or protrusions.

23. A connection adapter (100) according to any of the preceding claims, wherein the second adapter part (120) locks in the first adapter part (110) when the second adapter part is in the second position.

24. A connection adapter (100) according to any of the preceding claims, wherein the second adapter part (120) locks in the first adapter part (110) in a position intermediate between the first and the second position.

25. A connection adapter (100) according to any of the preceding claims, wherein the second adapter part (120) is arranged in a position intermediate between the first and second positions.

26. A connection adapter (100) according to claim 25, wherein a plurality of positions intermediate between the first and second positions is provided.

27. The use of a connection adapter (100) according to claims 1-26 in a fixed or portable disinfection machine, facility and/or device or tool.

28. The use of a connection adapter (100) according to claims 1-26 in the flushing of hollow tools in the medical, technological, manufacturing and scientific devices industry, e.g. in scientific laboratories, clean room environments, such as space technology or precise manufacturing grade clean rooms, as well as in the food and food manufacturing industry.

29. The use of a connection adapter (100) according to claims 1-26 with a variety and/or plurality of adapters for flushing media outlets (160).
